# EUROPEAN PATENT SPECIFICATION

(11) **EP 3 858 388 B1**
(45) Date of publication and mention of the grant of the patent: **03.07.2024**
(21) Application number: 21154942.3
(22) Date of filing: 28.09.2016
(51) Int. Cl.: A61K 48/00, C12N 15/00, C07H 21/04, A61K 35/17

(54) **CHIMERIC ANTIGEN RECEPTOR (CAR) T CELLS AS THERAPEUTIC INTERVENTIONS FOR GVHD**
T-ZELLEN MIT CHIMÄREM ANTIGENREZEPTOR (CAR) ALS THERAPEUTISCHE EINGRIFFE BEI GVHD
LYMPHOCYTES T DE TYPE RÉCEPTEUR D'ANTIGÈNE CHIMÉRIQUE (CAR) EN TANT QU'INTERVENTIONS THÉRAPEUTIQUES DE GVHD

(30) Priority: 28.09.2015 US 201562233908 P
(43) Date of publication of application: 04.08.2021
(62) Divisional of application: 16852441.1
(73) Proprietor: Regents of the University of Minnesota, Minneapolis, MN 55455-2020 (US)
(72) Inventor: BLAZAR, Bruce R., Golden Valley, Minnesota 55416 (US); FLYNN, Ryan P., Minneapolis, Minnesota 55455-2020 (US); PENNELL, Christopher A., Minneapolis, Minnesota 55455-2020 (US)
(74) Representative: Grund, Martin

(56) References cited:
- WO-A1-2011/060218
- WO-A2-2014/012001
- J. C. LEE ET AL: "In vivo Inhibition of Human CD19-Targeted Effector T Cells by Natural T Regulatory Cells in a Xenotransplant Murine Model of B Cell Malignancy", CANCER RESEARCH, vol. 71, no. 8, 12 April 2011 (2011-04-12) , pages 2871-2881, XP055109419, ISSN: 0008-5472, DOI: 10.1158/0008-5472.CAN-10-0552
- SYA N UKENA ET AL: "Isolation strategies of regulatory T cells for clinical trials: Phenotype, function, stability, and expansion capacity", EXPERIMENTAL HEMATALOGY, ELSEVIER INC, US, vol. 39, no. 12, 12 August 2011 (2011-08-12), pages 1152-1160, XP028112107, ISSN: 0301-472X, DOI: 10.1016/J.EXPHEM.2011.08.010 [retrieved on 2011-08-22]
- JETHWA HANNAH ET AL: "Use of gene-modified regulatory T-cells to control autoimmune and alloimmune pathology: Is now the right time?", CLINICAL IMMUNOLOGY, ELSEVIER, AMSTERDAM, NL, vol. 150, no. 1, 16 November 2013 (2013-11-16), pages 51-63, XP028671363, ISSN: 1521-6616, DOI: 10.1016/J.CLIM.2013.11.004

## Description

### TECHNICAL FIELD

This disclosure generally relates to immunology and, more specifically, to chimeric antigen receptor (CAR) T cell technology.

### BACKGROUND

Currently, therapies for autoimmune diseases and alloimmune diseases are limited, and typically treat the symptoms as opposed to the actual disease. Accordingly, novel therapies for autoimmune diseases and alloimmune diseases would be beneficial.

Lee et al., Cancer Research, 2011, 71(8):2871-2881 suggests a potential of CAR-modified natural T-regulatory cells as an approach for the treatment of autoimmune diseases. Different methods of treating Graft-vs-Host Disease (GVHD) comprising the use of Tregs are disclosed in Ukena et al., Experimental Hematalogy, 2011, 39(12):1152-1160, WO 2011/060218 A1, Jethwa et al., Clinical Immunology, 2013, 150(1):51-63, and WO 2014/012001 A2.

### SUMMARY

The invention is defined by the appended claims.

In one aspect, a pharmaceutical composition for use in treating graft-vs-host disease (GVHD) in a human patient, wherein the pharmaceutical composition comprises a therapeutically effective amount of a population of modified human regulatory T cells (Treg cells), wherein the human Treg cells are modified to express a nucleic acid sequence that encodes a chimeric antigen receptor (CAR) construct, wherein the CAR construct comprises an antigen binding domain, wherein the antigen binding domain is specific for CD19 is provided.

In some embodiments, the T cells are autologous to the human patient. In some embodiments, the T cells are allogeneic to the human patient.

In some embodiments, the modified T cells replicate in vivo in the human patient. In some embodiments, the modified T cells form memory T cells in the human patient against B cells, plasma cells or plasmablasts expressing a ligand recognized by the antigen binding domainIn some embodiments, the effective amount of T cells is between about 10^4 to about 10^9 cells per kg body weight of the human patient (e.g., between about 10^5 and about 10^6 cells per kg body weight of the human patient). In some embodiments, the modified T cells are administered intravenously to the human patient.

In some embodiments, the antigen binding domain is an antibody or an antigen-binding fragment thereof. In some embodiments, the antigen binding fragment is a Fab or scFv.

### DESCRIPTION OF DRAWINGS

Figure 1A is a scatter graph showing the frequency of CD45R B cells in the peripheral blood of mice having the genotype indicated on the x-axis. Each symbol represents a different mouse. The "***" denotes a statistically significant difference between groups with a p-value of <0.001.
Figure 1B is a scatter graph showing the frequency of mouse (m)CD19 B cells in the peripheral blood of mice having the genotype indicated on the x-axis. Each symbol represents a different mouse. The "***" denotes a statistically significant difference between groups with a p-value of <0.001.
Figure 1C is a scatter graph showing that B cell frequencies, as determined by expression of the human CD19 protein, were consistent with the values determined by the other B cell-specific markers, CD45R and mouse CD19. Each symbol represents a different mouse. The "***" denotes a statistically significant difference between groups with a p-value of <0.001.
Figure 1D is a graph showing the median fluorescence intensity (MFI), which was measured to determine the expression of human CD19 protein on mouse B cells. Each symbol represents a different mouse. The "***" denotes a statistically significant difference between groups with a p-value of <0.001.
Figure 2 is a line graph showing that mouse T cells retrovirally transduced with a construct encoding a human CD19-specific CAR expanded significantly in vitro under the appropriate culture conditions. The y axis shows the fold expansion in cell number over 5 days relative to the starting cell number on day 1.
Figure 3A depicts percent specific lysis, which means killing only of the human CD 19+ tumor targets, and not of the admixed human CD 19 negative tumor cells. The numbers of both cell types were determined 4 and 14 hours after the addition of varying numbers of CART-19 cells. The ratio of effector CART-19 cells to the human CD19+ tumor target cells (the E:T ratio) varied from 0 to 10.
Figure 3B is a bar graph showing there was no off-target cytotoxicity of CART-19 cells in vitro. This is evidenced by the similar numbers of TBL12 (human CD19 negative) tumor cells in wells containing varying numbers of CART-19 cells; TBL12 cells were enumerated 4 and 14 hours after the addition of CART-19 cells in vitro.
Figure 4A is a photograph showing that, in huCD19TG+/- mice, CAR T-19 cells (green) deplete B cells (red).
Figure 4B is a photograph showing that, in control huCD19TG+/- mice, CAR T cells (green) are present but did not expand to the extent shown in Figure 4A, and that B cells (red) are plentiful and have not been depleted.

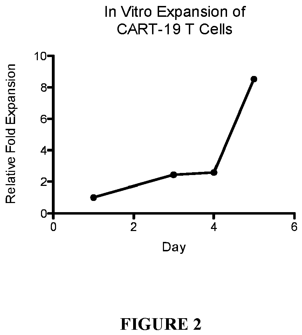

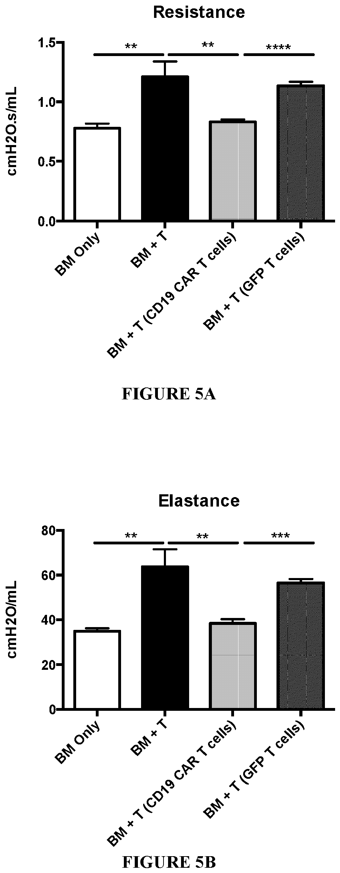

Figure 5A is a bar graph showing the pulmonary resistance in mice measured in intubated mice that were mechanically ventilated on day 60 post-transplant. Pulmonary function tests were measured by whole-body plethysmography using the Flexivent system (Scireq) on day 60 post-transplant. All mice received bone marrow. In the absence of supplemental T cells, the mice did not develop chronic GVHD and served as the bone marrow transplant (BMT) controls. Mice receiving BM+T cells had been given supplemental T cells to induce chronic GVHD. On day 28, indicated groups received no additional therapy or donor T cells that were transduced to express anti-CD19 ScFv CAR or a green fluorescent protein (GFP) control protein, expanded in vitro as per Figure 2, and then infused in vivo at a dose of 0.3 x 10^6 CAR-T cells or GFP-T cells. The high resistance is indicative of chronic GVHD which is ameliorated by CAR but not GFP T cells. **P≤0.01; ****P<0.0001.
Figure 5B is a bar graph showing the pulmonary elastance in mice after 60 days as per Figure 5A. The high elastance in chronic GVHD mice is indicative of loss of recoil properties which is restored by CAR-T cells but not GFP-T cells. **P ≤ 0.01; ***P ≤ 0.001.
Figure 5C is a bar graph showing the compliance after 60 days as per Figure 5A. The low compliance in chronic GVHD mice is indicative of stiff lungs, which is restored by CAR-T cells but not GFP-T cells. **P ≤ 0.01; ***P ≤ 0.001; ****P ≤ 0.0001.
Figure 6 is a scatter graph showing that hCD19+ cells persist in recipient mice at day 4 post transplantation (from CD4- lymphocytes).
Figure 7 is a graph showing survival of mice.
Figure 8 is a graph showing the weight of mice.
Figure 9 is a graph showing the clinical scores of mice after transplant.

### DETAILED DESCRIPTION

Immunotherapeutic methods are described herein that can be used to treat an autoimmune disease or an alloimmune disease in a human patient. Such a method typically includes administering a pharmaceutical composition to the human patient that includes an effective amount of modified human T cells. The modified T cells as used herein refer to T cells that have been modified to include a nucleic acid sequence that expresses and encodes a chimeric antigen receptor (CAR). These modified T cells oftentimes are referred to as CAR-T cells. As an alternative to CAR-T cells, modified human T cells can be used, wherein the modified human T cells refer to T cells in which the T cell receptor gene has been modified so as to recognize an antigen (or peptide) on B cells, plasma cells or plasmablasts.

CAR-T cells are known in the art and typically include a CAR construct. In addition to a nucleic acid encoding at least one antigen binding domain that is specific for any ligand that is present on B cells, plasma cells or plasmablasts, a typical CAR construct includes nucleic acids encoding a signal peptide (e.g., the signal peptide native to a scFv light chain, the signal peptide native to a scFv heavy chain), a hinge region (e.g., from CD8 alpha, CD3 or IgG1), a transmembrane domain (e.g., from CD8 alpha, CD3 zeta or CD28), a signal transmitting domain (e.g., from CD3 zeta or CD28) and, as necessary, a co-stimulatory signaling domain (e.g., from CD27, CD28 or OX40). See, for example, U.S. Patent Nos. 8,822,647 and 9,328,156. The antigen binding domains suitable for use in a CAR construct described herein are those that are useful for treating autoimmune diseases or alloimmune diseases.

Methods of making CAR constructs and CAR-T cells are known in the art. Methods of making CAR constructs typically include standard recombinant and molecular biology techniques. CAR constructs then can be introduced into T cells using transfection techniques that are known in the art. Alternatively, T cell receptor genes can be modified using, for example, zinc finger nucleases (see, e.g., US Patent No. 8,956,828).

The CAR construct is introduced into T cells using known methods. In some instances, the T cells are autologous to the patient (obtained from the patient, modified with the CAR construct, and introduced back into the patient), while in other instances, the T cells are allogeneic to the patient (obtained from a related or unrelated individual).

The methods described herein can be applied to autoimmune diseases and alloimmune diseases. Exemplary non-limiting examples of autoimmune diseases include chronic graft-vs-host disease (GVHD), lupus, arthritis, immune complex glomerulonephritis, goodpasture, uveitis, hepatitis, systemic sclerosis or scleroderma, type I diabetes, multiple sclerosis, cold agglutinin disease, Pemphigus vulgaris, Grave's disease, autoimmune hemolytic anemia, Hemophilia A, Primary Sjogren's Syndrome, thrombotic thrombocytopenia purrpura, neuromyelits optica, Evan's syndrome, IgM mediated neuropathy, cyroglobulinemia, dermatomyositis, idiopathic thrombocytopenia, ankylosing spondylitis, bullous pemphigoid, acquired angioedema, chronic urticarial, antiphospholipid demyelinating polyneuropathy, and autoimmune thrombocytopenia or neutropenia or pure red cell aplasias, while exemplary non-limiting examples of alloimmune diseases include allosensitization (see, for example, Blazar et al., 2015, Am. J. Transplant., 15(4):931-41) or xenosensitization from hematopoietic or solid organ transplantation, blood transfusions, pregnancy with fetal allosensitization, neonatal alloimmune thrombocytopenia, hemolytic disease of the newborn, sensitization to foreign antigens such as can occur with replacement of inherited or acquired deficiency disorders treated with enzyme or protein replacement therapy, blood products, and gene therapy.

Antigen binding domains that are specific for a ligand on B cells, plasma cells or plasmablasts are useful in the methods of treating autoimmune diseases or alloimmune diseases as described herein. For example, a CAR construct can contain an antigen binding domain that is specific for, without limitation, CD19, CD20, CD22, CD138, BCMA, CD319, CD10, CD24, CD27, CD38, or CD45R. In addition, a CAR construct can contain an antigen binding domain that is specific for, without limitation, an autoimmune specific antigen. Autoimmune specific antigens include, for example, the antigen that results in systemic lupus erythematosus (SLE), Graves' disease, celiac disease, diabetes mellitus type 1, rheumatoid arthritis (RA), sarcoidosis, Sjögren's syndrome, polymyositis (PM), and dermatomyositis (DM). See, for example, Ellebrecht et al., 2016, Science, 353:179-84.

The nucleic acid sequence of a representative CAR construct is shown in SEQ ID NO:1. The CAR construct shown in SEQ ID NO:1 has an antigen binding domain that is specific for CD19 (nt 1-8 10 of SEQ ID NO:1), however, it would be understood by a skilled artisan that a CAR construct expressing any number of antigen binding domains can be used in the methods described herein. For example, Uckun et al., 2011, Brit. J. Hematol., 153:15-23; US 2012/0141505; and US Patent Nos. 5,484,892; 5,573,924; 6,379,668; 7,744,877; 8,362,211; 9,023,999; and 9,034,324 describe the sequences of a number of antigen binding domains that can be used in compositions (e.g., CAR constructs) and methods as described herein.

It would be appreciated that the antigen binding domain can be, for example, an antigen binding domain from an immunoglobulin, or an alpha or a beta chain of a T cell receptor (TCR), or an antigen binding domain can be an antigen binding fragment (e.g., a scFv or a Fab). It also would be appreciated that a CAR construct can be designed to have elements that express the CAR constitutively or inducibly, thus providing further control over the therapeutic ability of the CAR-T cells.

As used herein, an effective amount of CAR-T cells refers to an amount that results in the desired therapeutic endpoint (e.g., a reduction, amelioration or elimination of symptoms, a reduction in or elimination of the autoantibodies or alloantibodies in the human patient) without resulting in toxicity to the human patient. Simply by way of example, an effective amount of CAR-T cells that are administered to a human patient can refer to between about 10^4 and about 10^9 CAR-T cells (e.g., between about 10^5 and about 10^6 CAR-T cells) per kg body weight of the human patient. It would be appreciated that a CAR-T cells typically are administered intravenously to a human patient.

As used herein, "treatment" refers to reversing, alleviating, or inhibiting the progress of an autoimmune or alloimmune disease, or one or more symptoms associated with such an autoimmune or alloimmune disease. It would be understood that the particular therapeutic endpoint(s) that determines whether or not treatment has been achieved (e.g., whether or not a patient has been treated) will depend upon whether the patient suffers from an autoimmune disease or an alloimmune disease as well as the particular type of autoimmune or alloimmune disease. In addition, the symptoms of many autoimmune and alloimmune diseases are widespread, nonspecific and/or diffuse, so the particular therapeutic endpoint(s) also depends upon the manifestation of the particular autoimmune or alloimmune disease (e.g., the tissue or organs affected, the severity or acuteness of the disease, or the coexistence of more than one disease) in each patient. Simply by way of example, Lee et al. (2015, Biol. Blood Marrow Transplant., 21:984-999) and Jagasia et al. (2015, Biol. Blood Marrow Transplant., 21:389-401) provide therapeutic and clinical guidelines for GVHD.

It would be appreciated by a skilled artisan that replication of the modified T cells in vivo in the human patient is indicative of successful treatment. In addition, it would be appreciated by a skilled artisan that the formation of memory T cells in the human patient also is a measure of success. It also would be appreciated by a skilled artisan that the modified T cells can persist in the human patient for a period of time of at least three months after administration (e.g., at least four months, at last five months, at least six months, at least seven months, at least eight months, at least nine months, at least ten months, at least eleven months, at least twelve months, at least two years, or at least three years after administration).

In addition to immunotherapy, the methods described herein also can be used for research in disruption of the B cell compartment by a cellular mechanism. For example, compounds can be screened to identify those that deplete B cells (e.g., so that T cells do not receive the proper signals to cause disease) or prevents or interferes with the cooperativity that takes place between B cells and T cells and is required for B cell-mediated antibody production. Representative compounds that can be screened include, without limitation, cells, drugs, small molecules, nucleic acids (e.g., DNAs, RNAs (e.g., interfering RNA (RNAi); e.g., shRNA or siRNA), protein, peptides, and small molecules.

### EXAMPLES

### Example 1-Optimization of a Mouse Model for Assessing On-Target / Off-Tumor Toxicity of Human CD19-Specific CAR T Cells

Mice were used that express a human CD19 transgene (huCD19TG) exclusively in healthy B cells. These cells should be killed upon transfer of human CD19-specific CAR T cells, and should measure on target/off tumor toxicity. This provides the closest animal model to the observed on target/off tumor toxicity observed in human clinical trials of human CD19-specific CAR T cells.

The first decision is whether to use mice that are huCD19TG homozygous (+/+) or hemizygous (+/-). The two key criteria are B cell frequencies and B cell levels of huCD19TG expression. Our goal is to choose the mouse that best models the human conditions. The huCD19TG mice are on the C57BL/6 background. The experiment below describes how B cell frequencies and huCD19TG expression levels were measured.

75-200 µl of blood per mouse was obtained via venipuncture of the facial vein. Blood was collected from wild type (huCD19TG^{-/-}), hemizygous (huCD19TG^{+/-}), and homozygous (CD19^{+/+}) mice, and collected in a 1.5 ml Eppendorf tubes containing the anticoagulant, heparin. Fluorophore-conjugated antibodies that were specific for B cell-specific proteins were added to 75 µl blood per mouse. The antibody specificities were mouse CD19 (a B cell specific marker), human CD19 (encoded by the transgene), and mouse CD45R (a B cell-specific marker). Samples were incubated for 15-30 minutes at room temperature in the dark. Red blood cells were lysed using 1X ACK lysis buffer. The remaining white bloods cells were washed two times in FACS buffer.

Each sample then was analyzed by flow cytometry to determine the frequencies of B cells in the lymphocyte populations. To do this, lymphocytes were selected based on their characteristic forward (FSC) and side scatter (SSC) light properties. Forward scatter measures size and side scatter measures intracellular complexity. The frequencies of lymphocytes that express mouse CD19, human CD19, and mouse CD45R were determined by dividing the number of antibody binding cells in the lymphocyte gate by the total number of cells in the lymphocyte gate.

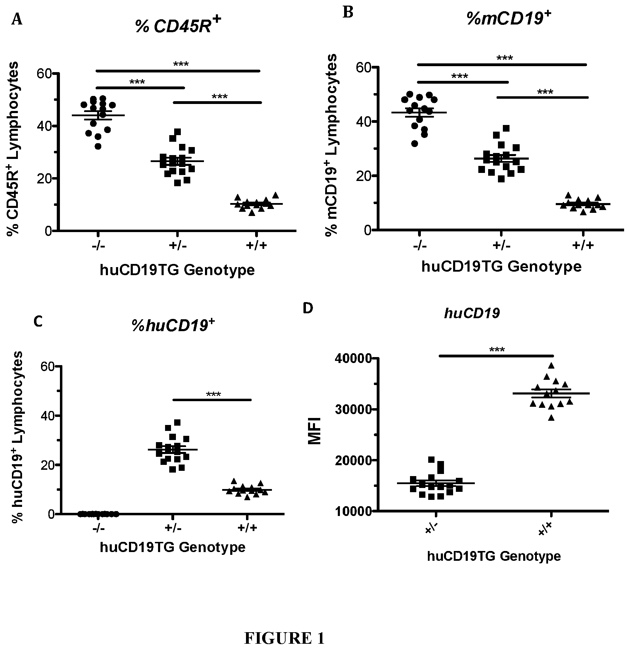

The data in Figures 1A, 1B, and 1C are presented as scatter grams in which the y axis depicts the frequency of antibody binding lymphocytes and the x axis displays the lymphocyte source. The data in Figure 1D show the relative levels of the human CD19 protein expressed on peripheral blood B cells from huCD19TG^{+/+} and huCD19TG^{+/-} mice. MFI is an abbreviation for median fluorescence intensity, a direct measure of specific antibody binding and, therefore, an indirect measure of the cell surface quantity of protein detected by the antibody (in this case, human CD19).

The average frequencies of B cells in peripheral blood lymphocytes in wildtype (-/-), huCD19 hemizygous (+/-), and huCD19 homozygous (+/+) mice are, respectively, 43%, 26%, and 10%. These frequencies are consistent when using either mouse CD45R or mouse CD19 to identify peripheral blood B cells. These frequencies also are consistent when using human CD 19 to identify peripheral B cells from huCD 19TG^{+/+} and huCD 19TG^{+/-} mice. It is noted that wild type mice (huCD19TG^{-/-}) do not express the human CD19 protein and so have no lymphocytes that are detectable using an anti-human CD19 antibody. Peripheral blood B cells from huCD19TG hemizygous (+/-) mice express about half the level of human CD19 as compared to peripheral blood B cells from huCD19TG homozygous (+/+) mice. This expression level correlates with there being twice as many copies of the huCD19TG in the genomes of homozygous versus hemizygous huCD19TG mice.

Based on data presented in Figure 1, it was decided to use huCD19TG hemizygous (+/-) mice, rather than huCD19TG homozygous (+/+) mice, as recipients of adoptively transferred human CD19-specific CAR T cells because the frequency of peripheral blood B cells in hemizygous mice more closely approximates the frequency of peripheral blood B cells in normal, non-transgenic mice. In tumor therapy experiments, the expression levels of human CD19 on tumors can be matched with the levels on peripheral blood B cells from huCD19TG hemizygous (+/-), in order to better model the human situation.

### Example 2-Infection of Primary Mouse Lymphocytes with a Retrovirus Encoding a Human CD19-Specific Chimeric Antigen Receptor (CAR) and Ex Vivo Expansion of Those Cells

The CAR construct, hCD19CAR, which has been used in clinical trials, was provided by Dr. Michael Jensen (University of Washington, Seattle). See, for example, SEQ ID NO:1, which contains a CD19RscFv portion (nt 1-810 of SEQ ID NO:1), an IgG4 hinge portion (nt 811-846 of SEQ ID NO:1), a CD28tm portion (nt 847-930 of SEQ ID NO:1), a 4-1BB portion (nt 931-1056 of SEQ ID NO:1), a Zeta portion (nt 1057-1392 of SEQ ID NO:1), a T2A portion (nt 1393-1464 of SEQ ID NO: 1), and a EGFRt portion (nt 1465-2538 of SEQ ID NO:1). A published protocol to expand and infect mouse T cells with the retrovirus was modified as follows.

### T cell enrichment prior to retroviral transduction

Magnetic beads were used to negatively enrich mouse splenocytes for conventional CD3⁺ alpha / beta T cells. Single cell suspensions of splenocytes were incubated with biotinylated antibodies specific for myeloid cells (CD 11b, CD11c), B cells (CD19, CD45R), NK cells (NK1), gamma / delta T cells, and T regulatory cells (CD25). Iron particles conjugated to streptavidin were added. Cells specifically bound by the biotinylated antibodies then were coated with iron particles. The tube containing the cell/iron particle cocktail was placed in a strong magnetic, and the unbound cells were removed. These "negatively enriched" cells contained between 91-95% CD3⁺ T cells. The in-house laboratory protocol was followed to expand and infect mouse T cells (see below).

### T cell expansion retroviral transduction

The amount of beads (Gibco by Life Technologies Dynabeads mouse T activator CD3/CD28) required to obtain a 2:1 bead:cell ratio was calculated. Beads were washed by diluting the required number of beads in ~3mL PBS + 2% FCS in a FACS or 15 mL tube. Suspended beads were placed into the MPC-50 magnet and beads were allowed to collect on the side of the tube for 1 min. Samples were aspirated with PBS + 2% FCS solution and the tube was removed from the magnet. Beads were resuspended in new PBS + 2% FCS4 and washed two additional times. After the last wash, beads were resuspended in up to 1 mL complete RPMI (DMEM). The washed, resuspended beads were added to T cells, making sure that the final concentration of cell is 2 x 10^6 cells/mL with a bead to cell ratio of 2:1. The cell solution was supplemented with 100 IU/mL rHuman IL-2. 2 mL of cell solution was added to each well in a 24-well tissue culture treated plate. Cells were incubated at 37°C with 5% CO₂ for 48 hr.

To prepare for spinoculation #1 (on day 0), a 24-well tissue culture plate was coated with 100 µg/mL RetroNectin in PBS. Samples were incubated at room temperature for 3 hr OR overnight at 4°C.

Transduction of murine T cells was performed with the viral supernatant by spinoculation #1 (on day 1) as follows. A volume from each condition was pipetted and pooled into an appropriate sized Falcon tube and activated T-cells were counted from day -1. Cells were gently spun (e.g., 1200 rpm for 10 minutes) and resuspended at 2 x 10^6 cells/mL in complete RPMI (DMEM) supplemented with 100 IU of rHuman IL-2. Prior to spinoculation, the RN/PBS solution was aspirated from RetroNectin-coated plates. The wells were washed and aspirated once more with 1 mL PBS. 1 mL (or dilutions) of previously fresh or thawed viral supernatant were added per well. Immediately after adding the viral supernatant, 1 mL of T cells was added. Each well contained a total volume of 2 mL with 2 x 10^6 T cells and viral supernatant. The plate(s) were immediately centrifuged at 2,000 g (2960 rpm) at 30°C for 1 h (first spinoculation). Cells were incubated at 37°C in 5% CO₂ overnight.

For spinoculation #2 (on day 2), media from each well was aspirated, taking care not to disturb the T cells at the bottom of the well. The amount of media removed was equal to the amount of virus added to the well on Day 1. Each well was replenished with 1 mL (or dilutions) of fresh or thawed viral supernatant. Plates were centrifuged at 2,000 g (2960 rpm) at 30°C for 1 h (second spinoculation). Cells were incubated at 37°C in 5% CO₂ overnight.

On day 3, 0.5-1 mL of media was removed from each well and 0.5-1 mL fresh complete RPMI (DMEM) media supplemented with 100 IU rHuman IL-2 was added back to each well. On days 4-7, T cell counts were obtained daily or every other day to maintain a T cell concentration of 1-2 x 10^6 cells/mL for optimal expansion and viability. 1-2 wells per condition were reserved to use for counting and not for eventual ACT. On day 5, transduction efficiency of T cells was analyzed by FACS. Cells were harvested as appropriate for functional assays. On day 7, transduction efficiency of T cells was analyzed by FACS. Optimal efficiency was generally observed between day 6 and day 7. Cells were harvested for functional assays, typically on the day of adoptive transfer.

As shown in Figure 2, these experiments demonstrated that the ex vivo retroviral transductions and expansion were successful.

### Example 3-Functionality of CAR T Cells In Vitro and Their Ability to Kill Human CD19+ Target Cells

The human CD19 gene, along with reporter genes encoding green fluorescent protein (GFP) and luciferase, were introduced into a mouse B cell tumor called TBL12. This derivative was designated TBL12.huCD19. Both the parental TBL12 and the derivative TBL12.huCD19 lines express mouse CD19, but only the derivative line, TBL12.huCD19, expresses human CD19 and GFP. Both of these proteins are detectable by flow cytometry.

Equal numbers of TBL12 and TBL12.huCD19 tumor cells along with varying numbers of CAR T cells taken on day 7 in the transduction protocol were combined in 24 well tissue culture plates. Cells were incubated together for either 4 or 14 hours at 37°C and then analyzed by flow cytometry as follows. A defined number of CountBright beads was added to a defined volume of cells in each FACS tube. Sorting was gated on mCD19+ targets (both TBL12 lines are mCD19+), and GFP+ and GFP- cells were quantitated (GFP+ = TBL12.hCD19; GFP- = TBL12). Triplicate measurements per condition were obtained.

Determining the percent specific lysis was calculated as follows. The total number of target cells / FACS tube was solved ("d"): a/b = c/d where a = number of bead events collected (5000), b = total number of beads/FACS tube (18800 beads based on 20 µl at 940 beads/µl); c = cell count in GFP+ or GFP- gated population; and d = total number of gated cells/FACS tube. The TBL12/TBL12.hCD19 ratios were calculated using the absolute numbers of gated cells ("d"). The percent specific lysis = (1 - [Control ratio/Exp ratio]) x 100, where the Control = TBL12/TBL12.hCD19 ratio in the 0:1 E:T sample and the Experimental = TBL12/TBL12.hCD19 ratios in the 1:1, 3:1, 10:1 E:T samples.

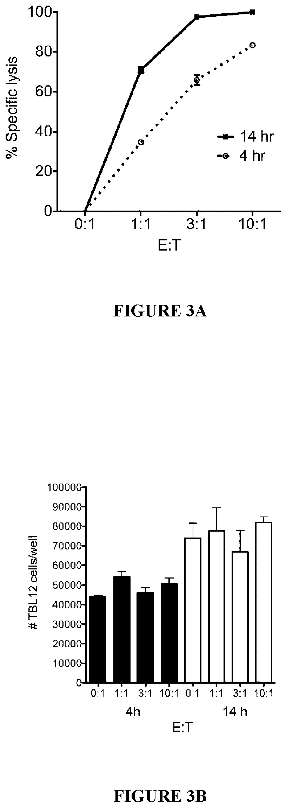

The data in Figure 3A are presented as the percent specific lysis (y axis) of TBL12.huCD19 target cells at varying ratios of effector (CAR T cells) to target cells (x axis). Data are shown as mean percent specific lysis (from triplicate samples) ± standard error of the mean. The data in Figure 3B are presented as the absolute number of TBL12 (parental, non-target) cells/well in the various test conditions. Black columns depict cell numbers after 4 hours of culture while open columns depict cell numbers after 14 hours of culture. All the E:T ratios were significantly different from controls (0:1) within a group (Figure 3A); all E:T ratios between groups are significantly different (Figure 3A); and there were no significant differences in TBL12 cell numbers among treatment groups within a given culture period (Figure 3B).

As shown in Figure 3A and 3B, the anti-human CAR T cells specifically kill human CD19-bearing tumor targets in vitro.

### Example 4-Functionality of CAR T Cells In Vivo and Their Ability to Kill Human CD19+ B Cells in huCD 19TG^{+/-} Mice

Anti-human CD19 CAR T cells (0.3 x 10^6), or control T cells transduced with a retrovirus encoding the reporter protein GFP (0.3 x 10^6), were injected intravenously into huCD19TG^{+/-} mice. After four days, mice were euthanized and their spleens removed and frozen in optimal cutting temperature tissue medium. Thin (10 µm) sections were cut on a cryostat, fixed, and stained with fluorophore-conjugated antibodies specific for endogenous B cells or the adoptively transferred T cells. Tissue images were captured by confocal microscopy at 20X magnification.

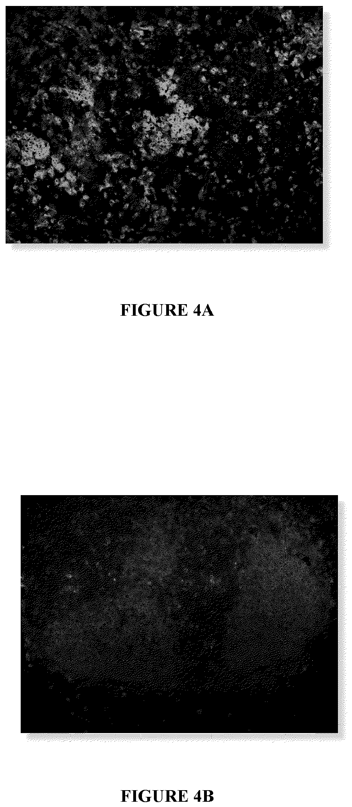

Figure 4A shows massive accumulation of CAR T cells (green) in the spleen of a huCD19TG^{+/-} mouse, and no detectable endogenous B cells (red). Figure 4B shows the spleen from a control huCD19TG^{+/-} mouse injected with T cells treated identically to the CAR T cells except the retrovirus encoded GFP, not the anti-human CD19 CAR. Endogenous B cells (red) are plentiful and the infiltrating T cells (green) are detectable but have not expanded to the extent seen in Figure 4A. The experimental results shown in Figures 4A and 4B demonstrate that CAR T cells engineered and expanded *ex vivo* kill human CD19⁺ B cells when transferred into huCD19TG^{+/-} mice. Thus, the huCD19TG^{+/-} model is suitable to assess on-target/off-tumor toxicity.

| Group | N | TBI | Conditioning Treatment | Recipients | BM | Splenocytes (Purified T cells) | Treatment |
|---|---|---|---|---|---|---|---|
| | | Day -1 | Day -2 and -3 | | Day 0 | Day 0 | D28 |
| 1 | 10 | 830X | 120 mg/kg Cytoxan | B10.BR | 10^7 B6 CD19 Tg | -- | -- |
| 2 | 10 | 830X | 120 mg/kg Cytoxan | B10.BR | 10^7 B6 CD19 Tg | 0.8 x 10^5 B6 WT | -- |
| 3 | 10 | 830X | 120 mg/kg Cytoxan | B10.BR | 10^7 B6 CD19 Tg | 0.8 x 10^5 B6 WT | CAR CD19 T cells iv |
| 4 | 10 | 830X | 120 mg/kg Cytoxan | B10.BR | 10^7 B6 CD19 Tg | 0.8 x 10^5 B6 WT | Transduced GFP T cells iv |

### Example 5-CAR CD19 Tregs Decrease aGVHD Severity and Incidence in hCD19 Mice

Tregs were purified from LN + SP of 115 wild type (WT) B6 mice using EasySep CD4 negative selection and CD25 positive selection. Analysis showed that the resulting product contained 99.9% CD4+ CD25+. Once purified, Tregs were activated for 4 days using plate-bound anti-CD3+ (2 µg) and CD28+ antibodies (4 µg).

Tregs were then transduced with either CAR hCD19-EGFR or Control RV-EGFR using routine methods. On day 5 post-transduction efficiency was low, so cells were transduced a second time using the same methods. On day 7 post-transduction, transduction efficiency was about 30% for both CAR hCD19 Treg cells and control Treg cells. At day 7 post-transduction, the purity was >93% for both groups, and the yield was 26.75 million CAR hCD19 Treg cells and 22.5 million control Treg cells.

| Group | N | Donor | Recipient | X-Ray | BM | T-cell Dose | Treg Dose |
|---|---|---|---|---|---|---|---|
| 1 | 5 | BALB/c | hCD19-tg B6 | 1100 | 10^7 | 0 | 0 |
| 2 | 5 | BALB/c | hCD19-tg B6 | 1100 | 10^7 | 2.5 x 10^6 | 0 |
| 3 | 5 | BALB/c | hCD19-tg B6 | 1100 | 10^7 | 2.5 x 10^6 | 1.25 x 10^6 Control RV Tregs |
| 4 | 5 | BALB/c | hCD19-tg B6 | 1100 | 10^7 | 2.5 x 10^6 | 1.25 x 10^6 hCD19-CAR Tregs |

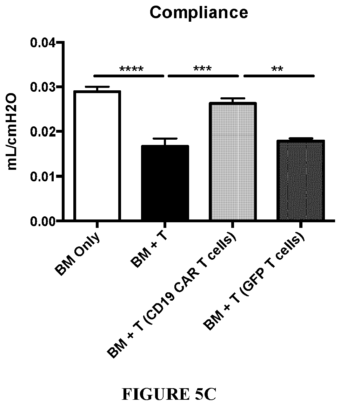

Figure 5 shows pulmonary function after 60 days; Figure 5A shows resistance of mice without or with chronic GVHD, and chronic GVHD mice treated on day 28 with CAR-T cells or GFP-T cells. High resistance indicative of chronic GVHD was reversed by CAR-T cells but not GFP-T cells; Figure 5B shows elastance of transplantation mice without or with chronic GVHD, and chronic GVHD mice treated on day 28 with CAR-T cells or GFP-T cells. High elastance indicative of chronic GVHD was reversed by CAR-T cells but not GFP-T cells; and Figure 5C shows low compliance of transplantation mice without or with chronic GVHD, and chronic GVHD mice treated on day 28 with CAR-T cells or GFP T-cells. Low compliance indicative of chronic GVHD was reversed by CAR-T cells but not GFP-T cells.

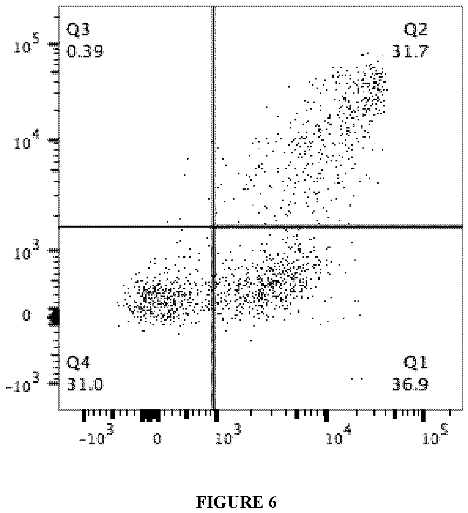

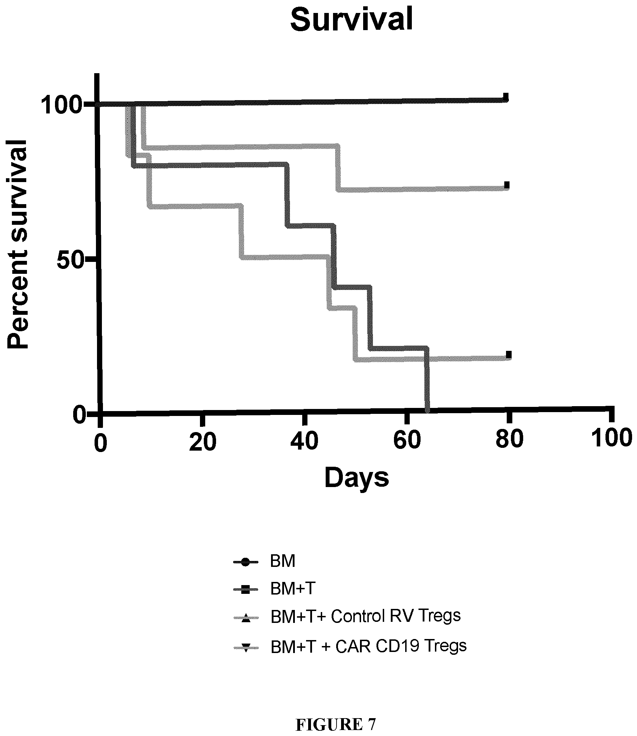

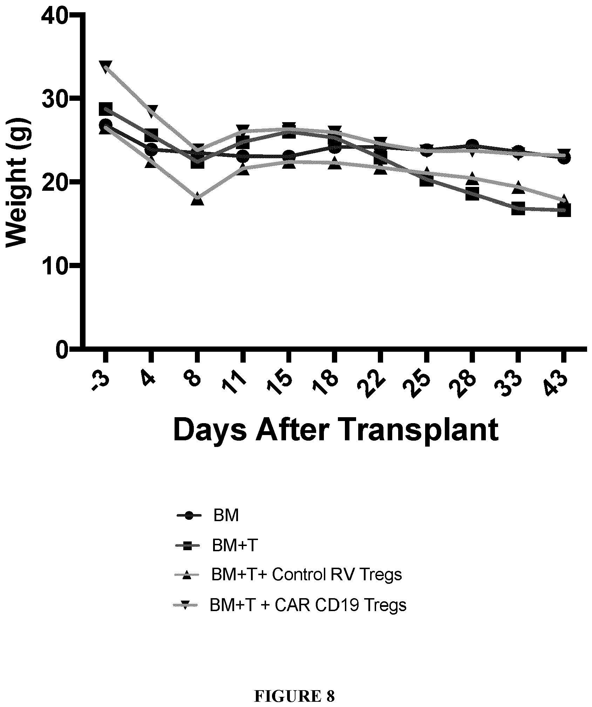

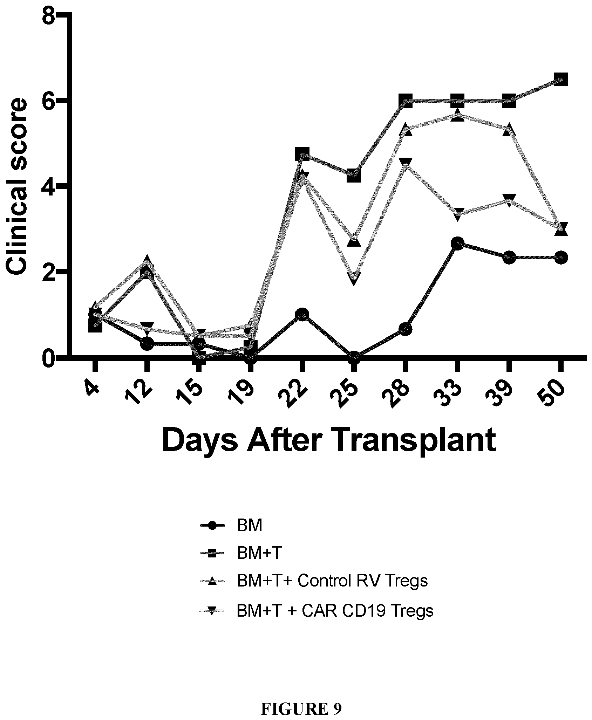

Figure 6 shows that hCD19+ cells persist in recipient mice at 4 days post-transplantation (from CD4- lymphocytes). Figure 7 shows that the addition of CAR CD19 Treg cells significantly improved survival of transplanted mice compared to mice transplanted with only bone marrow and T cells or mice transplanted with bone marrow and control Treg cells. Figure 8 shows that the weight loss of transplanted mice were similar, irrespective of whether T cells and/or CAR CD 19 Treg cells were delivered to the mice. Significantly, Figure 9 shows that mice transplanted with bone marrow, T cells and CAR CD 19 Treg cells exhibited a better clinical score than mice transplanted with bone marrow alone and a clinical score similar to mice transplanted with bone marrow and T cells or bone marrow, T cells and control Treg cells. Despite the fact that CAR-T cells, but not GFP-T cells, caused weight loss and clinical findings consistent with a cytokine release syndrome in CD19 heterozygous mice, unexpectedly, neither CAR-T cells nor GFP-T cells adversely affected survival.

## Claims

1. A pharmaceutical composition for use in treating graft-vs-host disease (GVHD) in a human patient, wherein the pharmaceutical composition comprises a therapeutically effective amount of a population of modified human regulatory T cells (Treg cells), wherein the human Treg cells are modified to express a nucleic acid sequence that encodes a chimeric antigen receptor (CAR) construct, wherein the CAR construct comprises an antigen binding domain, wherein the antigen binding domain is specific for CD 19.

2. The composition for the use of claim 1, wherein the Treg cells are autologous to the human patient.

3. The composition for the use of claim 1, wherein the Treg cells are allogeneic to the human patient.

4. The composition for the use of any of the preceding claims, wherein the modified Treg cells replicate in vivo in the human patient.

5. The composition for the use of any of the preceding claims, wherein the modified Treg cells form memory T cells in the human patient against B cells, plasma cells or plasmablasts expressing a ligand recognized by the antigen binding domain.

6. The composition for the use of any of the preceding claims, wherein the effective amount of Treg cells is between about 10^4 to about 10^9 cells per kg body weight of the human patient.

7. The composition for the use of any of the preceding claims, wherein the effective amount of Treg cells is between about 10^5 and about 10^6 cells per kg body weight of the human patient.

8. The composition for the use of any of the preceding claims, wherein the antigen binding domain is an antibody or an antigen-binding fragment thereof.

9. The composition for the use of claim 8, wherein the antigen binding fragment is a Fab or scFv.

10. The composition for the use of any of the preceding claims, wherein the modified Treg cells are to be administered intravenously to the human patient.

## Patentansprüche

1. Eine pharmazeutische Zusammensetzung zur Verwendung bei der Behandlung von Graft-vs-Host-Disease (GVHD) in einem menschlichen Patienten, wobei die pharmazeutische Zusammensetzung eine therapeutisch effektive Menge einer Population von modifizierten humanen regulatorischen T-Zellen (Treg-Zellen) umfasst, wobei die humanen Treg-Zellen modifiziert sind, um eine Nukleinsäuresequenz zu exprimieren, die ein chimäres Antigenrezeptor (CAR) -konstrukt kodiert, wobei das CARkonstrukt eine Antigenbindedomäne umfasst, wobei die Antigenbindedomäne spezifisch ist für CD19.

2. Die Zusammensetzung zur Verwendung nach Anspruch 1, wobei die Treg-Zellen autolog in Bezug auf den menschlichen Patienten sind.

3. Die Zusammensetzung zur Verwendung nach Anspruch 1, wobei die Treg-Zellen allogen in Bezug auf den menschlichen Patienten sind.

4. Die Zusammensetzung zur Verwendung nach einem der vorhergehenden Ansprüche, wobei die modifizierten Treg-Zellen in vivo in dem menschlichen Patienten replizieren.

5. Die Zusammensetzung zur Verwendung nach einem der vorhergehenden Ansprüche, wobei die modifizierten Treg-Zellen Memory T-Zellen in dem menschlichen Patienten gegen B-Zellen, Plasmazellen oder Plasmablasten bilden, die einen Liganden exprimieren, der von der Antigenbindedomäne erkannt wird.

6. Die Zusammensetzung zur Verwendung nach einem der vorhergehenden Ansprüche, wobei die effektive Menge der Treg-Zellen zwischen etwa 10⁴ und etwa 10⁹ Zellen pro kg Körpergewicht des menschlichen Patienten ist.

7. Die Zusammensetzung zur Verwendung nach einem der vorhergehenden Ansprüche, wobei die effektive Menge von Treg-Zellen zwischen etwa 10⁵ und etwa 10⁶ Zellen pro kg Körpergewicht des menschlichen Patienten ist.

8. Die Zusammensetzung zur Verwendung nach einem der vorhergehenden Ansprüche, wobei die Antigenbindedomäne ein Antikörper oder ein Antigen-bindendes Fragment davon ist.

9. Die Zusammensetzung zur Verwendung nach Anspruch 8, wobei das Antigen-bindende Fragment ein Fab oder ein scFv ist.

10. Die Zusammensetzung zur Verwendung nach einem der vorhergehenden Ansprüche, wobei die modifizierten Treg-Zellen intravenös an den menschlichen Patienten zu verabreichen sind.

## Revendications

1. Composition pharmaceutique pour une utilisation dans le traitement d'une maladie greffon contre hôte (GVHD) chez un patient humain, dans laquelle la composition pharmaceutique comprend une quantité thérapeutiquement efficace d'une population de lymphocytes T régulateurs humains modifiés (lymphocytes Treg), dans laquelle les lymphocytes Treg humains sont modifiés pour exprimer une séquence d'acide nucléique qui code pour une construction de récepteur antigénique chimérique (CAR), dans laquelle la construction CAR comprend un domaine de liaison à l'antigène, dans laquelle le domaine de liaison à l'antigène est spécifique à CD19.

2. Composition pour l'utilisation selon la revendication 1, dans laquelle les lymphocytes Treg sont autologues au patient humain.

3. Composition pour l'utilisation selon la revendication 1, dans laquelle les lymphocytes Treg sont allogéniques vis-à-vis du patient humain.

4. Composition pour l'utilisation selon l'une quelconque des revendications précédentes, dans laquelle les lymphocytes Treg modifiés se répliquent in vivo chez le patient humain.

5. Composition pour l'utilisation selon l'une quelconque des revendications précédentes, dans laquelle les lymphocytes Treg modifiés forment des lymphocytes T mémoires chez le patient humain contre des lymphocytes B, des plasmocytes ou des plasmoblastes exprimant un ligand reconnu par le domaine de liaison à l'antigène.

6. Composition pour l'utilisation selon l'une quelconque des revendications précédentes, dans laquelle la quantité efficace de lymphocytes Treg est comprise entre environ 10^4 à environ 10^9 cellules par kg de masse corporelle du patient humain.

7. Composition pour l'utilisation selon l'une quelconque des revendications précédentes, dans laquelle la quantité efficace de lymphocytes Treg est comprise entre environ 10^5 et environ 10^6 cellules par kg de masse corporelle du patient humain.

8. Composition pour l'utilisation selon l'une quelconque des revendications précédentes, dans laquelle le domaine de liaison à l'antigène est un anticorps ou un fragment de liaison à l'antigène de celui-ci.

9. Composition pour l'utilisation selon la revendication 8, dans laquelle le fragment de liaison à l'antigène est un Fab ou scFv.

10. Composition pour l'utilisation selon l'une quelconque des revendications précédentes, dans laquelle les lymphocytes Treg modifiés sont à administrer par voie intraveineuse au patient humain.
